(19) Europäisches Patentamt
European Patent Office
Office européen des brevets

(11) Publication number: 0 232 080 B1

(12) EUROPEAN PATENT SPECIFICATION

(45) Date of publication of patent specification: 16.05.90

(51) Int. Cl.$^5$: C 07 C 275/54, A 01 N 47/34

(21) Application number: 87300552.4

(22) Date of filing: 22.01.87

(54) Benzoylurea compounds and their production and use.

(30) Priority: 28.01.86 JP 17323/86

(43) Date of publication of application: 12.08.87 Bulletin 87/33

(45) Publication of the grant of the patent: 16.05.90 Bulletin 90/20

(84) Designated Contracting States: BE CH DE ES FR GB IT LI NL

(56) References cited:
EP-A-0 088 343
GB-A-2 168 702
US-A-4 310 548

(73) Proprietor: SUMITOMO CHEMICAL COMPANY, LIMITED
Kitahama 4-chome 5-33
Chuo-ku Osaka 541 (JP)

(72) Inventor: Ohsumi, Tadashi
7-10-202, Kawazoecho
Nishinomiya-shi (JP)
Inventor: Yano, Toshihiko
17-11 Shinasahigaoka
Ikoma-shi (JP)
Inventor: Tanabe, Yoo
10-3-342, Sonehigashinocho-2-chome
Toyonaka-shi (JP)
Inventor: Takada, Yoji
Sumitomo Kagaku Mefuryo Mefu-2-chome
Takarazuka-shi (JP)

(74) Representative: Harrison, David Christopher et al
MEWBURN ELLIS & CO 2/3 Cursitor Street
London EC4A 1BQ (GB)

EP 0 232 080 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to novel benzoylurea compounds, their production and use.

Japanese Patent Publication No. 18255/1977 discloses benzoylurea compounds effective against insects, and among these compounds, Diflubenzuron is already on the market. Also, from Japanese Patent Application Kokai (Laid-open) No. 126460/1982 and Japanese Patent Publication No. 53786/1982, it is well known that Teflubenzuron and Chlorfluazuron, described in the respective patents, have an insecticidal activity.

These compounds, however, are not always said to be satisfactory in that their activity is insufficient and their relatively high cost is a problem.

An object of the present invention is to provide a novel and more superior insecticide and its production.

In view of the above, we studied extensively the aniline portion of benzoylurea compounds, and as a result, found that the benzoylurea compounds of the present invention have extremely high insecticidal activity against the larvae of insects, their insecticidal activity being higher than that of the well-known homologues, and besides that they can be produced at relatively low costs.

The present invention provides a benzoylurea compound, useful as insecticide, represented by the formula (I) (hereinafter referred to as "present compound"),

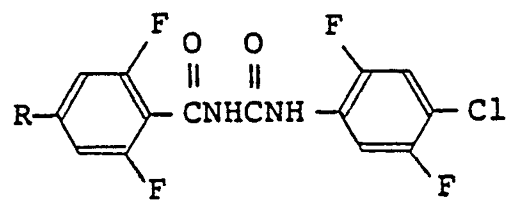
(I)

wherein R is a hydrogen atom or a fluorine atom.

A method for producing the present compounds will be illustrated in detail.

The present compounds represented by the formula (I) can be produced by the methods described below.

Method A:

A benzoylisocyanate compound represented by the formula (II),

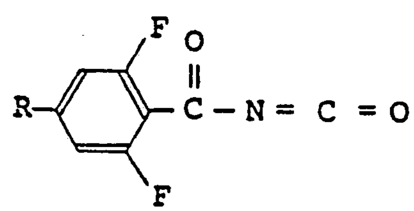
(II)

wherein R has the same meaning as described above, is treated with 2,5-difluoro-4-chloroaniline represented by the formula (III),

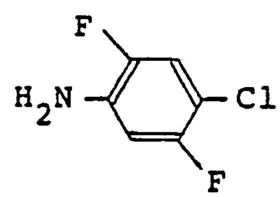
(III)

Method B:

A benzamide compound represented by the formula (IV),

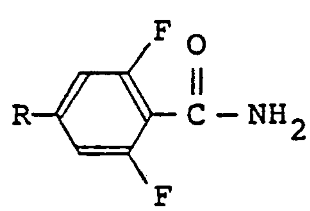
(IV)

wherein R has the same meaning as described above, is treated with an isocyanate compound represented by the formula (V),

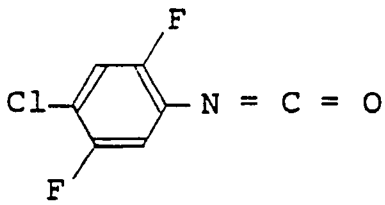 (V)

In the methods A and B, it is preferred to carry out the reaction in the presence of an inert solvent.

Preferred solvents include hydrocarbons such as benzene, toluene, xylene, chlorobenzene, carbon tetrachloride, chloroform, methylene chloride, 1,2-dichloroethane, nitromethane, etc.; ethers such as diethyl ether, tetrahydrofuran, dioxane, etc.; ketones such as acetone, methyl ethyl ketone, methyl isobutyl ketone, etc.; polar organic solvents such as dimethyl sulfoxide, dimethylformamide, sulfolane, etc.; and mixtures of two or more of these solvents.

The reaction in the methods A and B can generally be carried out at atmospheric pressure, and generally, it comes to an end in 1 to 50 hours. It is preferred in general that the materials for the reaction are used in equimolar amounts, but one of them may be used in excess.

The reaction temperature in the method A is not particularly limited, but it is a temperature, generally, between 0°C and 80°C, more generally between room temperature and 60°C.

The reaction temperature in the method B is not particularly limited, but it is a temperature, generally, between room temperature and 160°C, more generally between 80°C and 130°C.

The present compounds thus obtained may be purified if necessary by means such as column chromatography, recrystallization, etc.

Both the benzoylisocyanate compound represented by the formula (II) and the benzamide compound represented by the formula (IV), which are used in the methods of the present invention, are well-known compounds. The aniline compound represented by the formula (III) can be produced, for example, by the method shown in reference examples described later. Further, the isocyanate compound represented by the formula (V) can easily be produced from the aniline compound (III) and phosgene according to the usual method.

When the present compounds are used as insecticides, they may be used as such without adding any other ingredient. Generally, however, they are formulated into insecticide compositions containing an effective amount thereof as active ingredient, and an inert carrier or diluent. Thus, they may be formulated into emulsifiable concentrates, wettable powders, dusts, granules, oil sprays, aerosols, heating fumigants (e.g. mosquito coils, electric mosquito mats), fottings, non-heating fumigants, poisonous baits, etc. by mixing with solid carriers, liquid carriers, gaseous carriers, surface active agents, other auxiliaries for formulation, baits, etc., or impregnating into bases such as mosquito coil carriers, mats, etc.

These preparations may contain 0.01 to 95% by weight of the present compound as an active ingredient.

The solid carriers used for formulation include for example fine powders or granules of kaolin clay, attapulgite clay, bentonite, terra alba, pyrophyllite, talc, diatomaceous earth, calcite, corn stalk powder, walnut shell powder, urea, ammonium sulfate, synthetic hydrated silicon dioxide and the like. The liquid carriers include for example aliphatic hydrocarbons (e.g. kerosene), aromatic hydrocarbons (e.g. benzene, toluene, xylene, methylnaphthalene), halogenated hydrocarbons (e.g. dichloroethane, trichloroethylene, carbon tetrachloride), alcohols (e.g. methanol, ethanol, isopropanol, ethylene glycol, cellosolve), ketones (e.g. acetone, methyl ethyl ketone, cyclohexanone, isophorone), ethers (e.g. diethyl ether, dioxane, tetrahydrofuran), esters (e.g. ethyl acetate), nitriles (e.g. acetonitrile, isobutyronitrile), acid amides (e.g. dimethylformamide, dimethylacetamide), dimethyl sulfoxide, vegetable oils (e.g. soybean oil, cotton seed oil) and the like. The surface active agents used for emulsification, dispersion, wetting, etc. include for example anionic surface active agents such as the salt of alkyl sulfates, alkyl(aryl)sulfonates, dialkyl sulfosuccinates, the salt of polyoxyethylene alkylaryl ether phosphoric acid ester, naphthalenesulfonic acid/formalin condensates, etc., and nonionic surface active agents such as polyoxyethylene alkyl ether, polyoxyethylene polyoxypropylene block copolymers, sorbitan fatty acid esters, polyoxyethylene sorbitan fatty acid esters, etc.

The auxiliaries for formulation such as adhesive agents, dispersing agents, etc. include for example lignosulfonates, alginates, polyvinyl alcohol, gum arabic, molasses, casein, gelatin, CMC (carboxymethyl cellulose), pine oil, agar, etc. The stabilizers include for example alkyl phosphates [e.g. PAP (isopropyl acid phosphate), TCP (tricresyl phosphate)], vegetable oils, epoxidized oils, the foregoing surface active agents, antioxidants (e.g. BHT, BHA), fatty acid salts (e.g. sodium oleate, calcium stearate), fatty acid esters (e.g. methyl oleate, methyl stearate) and the like.

The preparations obtained are used as such or as aqueous dilute solutions. Also, they may be used in mixture with other insecticides, acaricides, nematocides, fungicides, herbicides, plant growth regulators, fertilizers, soil improvers and the like.

When the present compounds are used as insecticides, their dosage rate is generally 5 to 500 g per 10 ares. When emulsifiable concentrates, wettable powders, etc. are used as aqueous dilute solutions, the application concentration of the compounds is generally 10 to 1000 ppm. Dusts, granules, oil sprays, etc. are used as they are without dilution.

The present invention will be illustrated in more detail with reference to the following production examples, reference examples, formulation examples and test examples, but it is not of course limited to these examples.

First, production examples of the present compounds will be shown.

Production example 1

94 Milligrams of 2,5-difluoro-4-chloroaniline was dissolved in 5 ml of toluene, and to this solution, a solution of 100 mg of 2,6-difluorobenzoylisocyanate in 5 ml of toluene was added dropwise with icecooling and stirring. After completion of the addition, stirring was continued overnight at room temperature, and 10 ml of n-hexane was added to precipitate crystals. The crystals were filtered off and dried to obtain 170 mg of N-2,6-difluorobenzoyl-N′-2,5-difluoro-4-chlorophenylurea [hereinafter referred to as present compound (I)] as white crystals (yield, 90%; m.p., 201.6°C).

Production example 2

A mixture of 175 mg of 2,4,6-trifluorobenzamide, 190 mg of 2,5-difluoro-4-chlorophenylisocyanate and 20 ml of xylene was heated under reflux for 24 hours. After completion of the reaction, the reaction solution was cooled, and precipitated crystals were filtered off and recrystallized from acetone to obtain 133 mg of N-2,4,6-trifluorobenzoyl-N′-2,5-difluoro-4-chlorophenylurea [hereinafter referred to as present compound (2)] as white crystals (yield, 63%; m.p., 222.5°C).

Production of the materials will be shown in reference examples.

Reference example 1

8.00 Grams of 2,5-difluoroacetanilide was dissolved in 30 ml of 1,2-dichloroethane, and to this solution, a solution of 8.21 g of sulfuryl chloride in 10 ml of 1,2-dichloroethane was added dropwise under reflux with stirring. After completion of the addition, reaction was continued under reflux for 4 hours, and the reaction solution was cooled. n-Hexane was added to precipitate crystals. The crystals were filtered off and dried to obtain 8.64 g of 2,5-difluoro-4-chloroacetanilide as white crystals (yield, 90%).

PMR ($CDCl_3$):

δ (ppm) 9.0—8.0 (1H, broad), 8.40 (1H, dd, J=6.8, 10.2Hz), 7.19 (1H, dd, J=6.4, 10.2Hz), 2.22 (3H, s).

Reference example 2

A mixture of 7.90 g of 2,5-difluoro-4-chloroacetanilide, 4.30 g of potassium hydroxide, 10 ml of water and 30 ml of methanol was heated under reflux for 2 hours with stirring. The resulting solution was poured into ice water to precipitate crystals. The crystals were filtered off, washed with water and dried to obtain 5.02 g of 2,5-difluoro-4-chloroaniline (yield, 80%; m.p., 76.3°C).

PMR ($CDCl_3$):

δ (ppm) 7.02 (1H, dd, J=6.4, 11.0Hz), 6.56 (1H, dd, J=8.0, 10.0Hz), 4.5—3.0 (2H, broad).

Formulation examples will be shown. In the examples, the present compounds are shown by compound number in the production examples, and parts are by weight.

Formulation example 1

0.2 Part of the present compound (1), 2 parts of cyclohexanone and 97.8 parts of kerosene are mixed to obtain an oil spray.

Formulation example 2

Ten parts of each of the present compounds (1) and (2), 14 parts of polyoxyethylene styrylphenyl ether, 6 parts of calcium dodecylbenzenesulfonate, 35 parts of xylene and 35 parts of dimethylformamide are well mixed to obtain an emulsifiable concentrate of each compound.

Formulation example 3

20 Parts of the present compound (2), 10 parts of Fenitrothion, 3 parts of calcium lignosulfonate, 2 parts of sodium lauryl sulfate and 65 parts of synthetic hydrated silicon dioxide are well pulverized and mixed to obtain a wettable powder.

Formulation example 4

One part of the present compound (1), 2 parts of Carbaryl, 87 parts of kaolin clay and 10 parts of talc are well pulverized and mixed to obtain a dust.

Formulation example 5

Five parts of the present compound (2), 1 part of synthetic hydrated silicon dioxide, 2 parts of calcium lignosulfonate, 30 parts of bentonite and 62 parts of kaolin clay are well pulverized and mixed, well kneaded wth water, granulated and dried to obtain a granule.

That the present compounds are useful as an active ingredient for insecticides will be shown by test examples. In the examples, the present compounds are shown by the compound number in the production examples, and compounds used as a compound shown by the compound symbols in Table 1.

Table 1

| Compound symbol | Structural formula | Name |
|---|---|---|
| (A) | 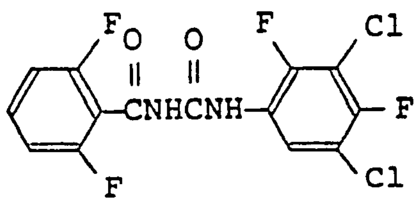 | Teflubenzuron |
| (B) | 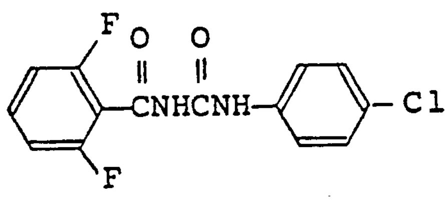 | Diflubenzuron |
| (C) | 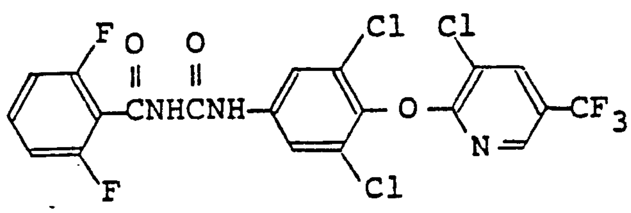 | Chlorfluazuron |
| (D) | 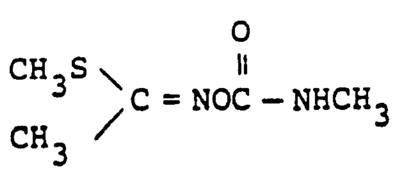 | Methomyl |

Test example 1

The emulsifiable concentrates of the following present compounds and controls obtained according to Formulation example 2 were each diluted with water to a prescribed concentration, and 2 ml each of the dilute solutions was impregnated into 13 g of artificial diet for tobacco cutworm (*Spodoptera litura*). The diet was then placed in a polyethylene cup of 11 cm in diameter, and 10 fourth instar larvae of tobacco cutworms were liberated therein. After six days, the dead and alive of the larvae were examined to obtain $LC_{50}$ value (50% lethal concentration) (two replications). The results are shown in Table 2.

Table 2

| Test compound | $LC_{50}$ (ppm) |
|---|---|
| (1) | 0.40 |
| (2) | 0.31 |
| (A) | 0.60 |
| (B) | 6.8 |
| (C) | 1.0 |
| (D) | 8.0 |

Test example 2

The emulsifiable concentrates of the following present compounds and controls obtained according to Formulation example 2 were each diluted with water to a concentration of 0.01 ppm, and 100 ml of the dilute solution was charged in a 180 ml polyethylene cup. Thereafter, 20 last instar larvae of northern house mosquitoes (*Culex pipiens pallens*) were liberated in the cup and bred until emergence to obtain an emergence rate (two replications). The results are shown in Table 3.

Table 3

| Test compound | Emergence rate (%) |
|---|---|
| (1) | 0 |
| (2) | 0 |
| Untreated | 90 |

Test example 3

The emulsifiable concentrates of the following present compounds and controls obtained according to Formulation example 2 were each diluted with water to a prescribed concentration, and 1 ml each of the dilute solutions was impregnated into 5 g of artificial diet for rice stem borer (*Chilo suppressalis*) which had been previously prepared in a polyethylene cup of 5.5 cm in diameter. Thereafter, 10-day old larvae (10 insects) of rice stem borers were liberated in the cup. After 8 days, the dead and alive of the larvae were examined to obtain $LC_{50}$ value (50% lethal concentration) (two replications). The results are shown in Table 4.

Table 4

| Test compound | $LC_{50}$ (ppm) |
|---|---|
| (1) | 0.61 |
| (2) | 0.60 |
| (A) | 1.2 |
| (B) | 6.5 |
| (C) | 3.5 |
| (D) | 50 |

Test example 4

The emulsifiable concentrates of the following present compounds and controls obtained according to Formulation example 2 were each diluted with water to a prescribed concentration, and each dilute solution was sprayed onto cabbage cultivated in pots which had elapsed one month after setting at a rate of 30 ml/3 pots on a turn table. After air-drying, fourth instar larvae of tobacco cutworms (*Spondoptera litura*) were liberated at a rate of 5 insects/pot. After seven days, the dead and alive of the larvae were examined to obtain a mortality (three replications). The results are shown in Table 5.

Table 5

| Test compound | Mortality (%) | |
|---|---|---|
| | 0.25 ppm | 0.125 ppm |
| (1) | 100 | 75 |
| (2) | 100 | 80 |
| (A) | 93 | 40 |
| (B) | 0 | 0 |
| (C) | 37 | 0 |
| (D) | 0 | 0 |
| Untreated | 0 | |

**Claims**

1. A benzoylurea compound represented by the formula,

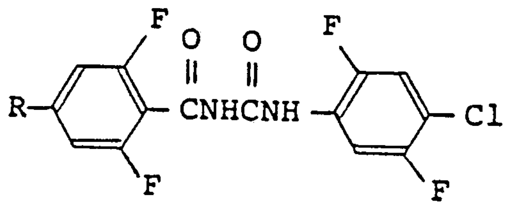

wherein R is a hydrogen atom or a fluorine atom.

2. The benzoylurea compound according to Claim 1, wherein R is a hydrogen atom.

3. The benzoylurea compound according to Claim 1, wherein R is a fluorine atom.

4. A method for producing a benzoylurea compound represented by the formula,

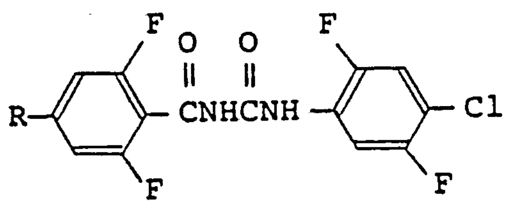

wherein R is a hydrogen atom or a fluorine atom, which comprises reacting a bezoylisocyanate compound represented by the formula,

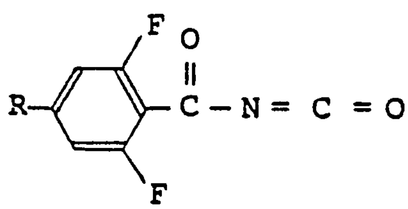

wherein R is as defined above, with an aniline compound represented by the formula,

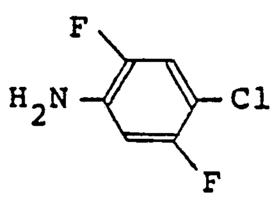

5. A method for producing a benzoylurea compound represented by the formula,

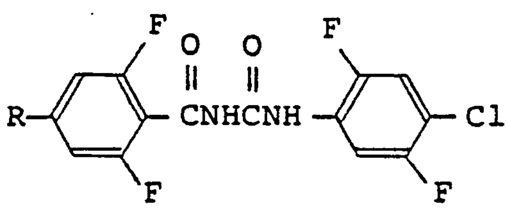

wherein R is a hydrogen atom or a fluorine atom, which comprises reacting a benzamide compound represented by the formula,

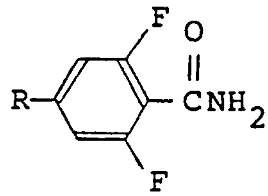

wherein R is as defined above, with an isocyanate compound represented by the formula,

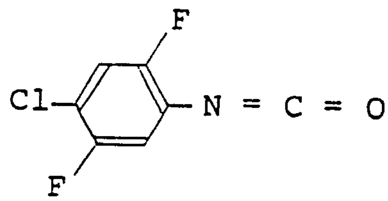

6. An insecticidal composition which comprises a benzoylurea compound according to Claim 1 and an inert carrier or diluent.

7. The insecticidal composition according to Claim 6, wherein the benzoylurea compound is represented by the formula,

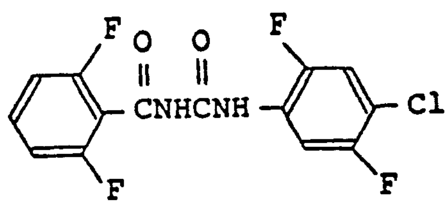

8. The insecticidal composition according to Claim 6, wherein the benzoylurea compound is represented by the formula,

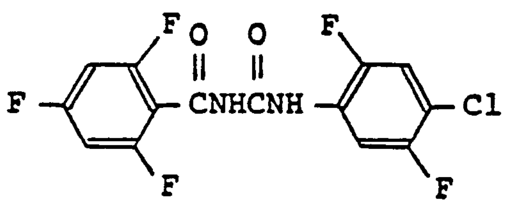

9. A method for controlling or exterminating insects which comprises applying an insecticidally effective amount of the benzoylurea compound according to Claim 1 to the insects.

10. Use of an insecticidal composition according to Claim 6 as an insecticide.

**Patentansprüche**

1. Eine Benzoylharnstoff-Verbindung der Formel

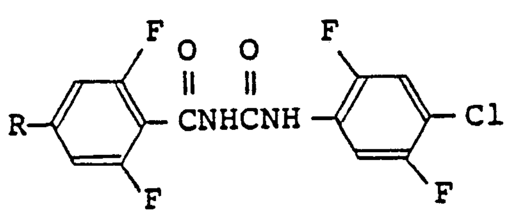

in der R ein Wasserstoff- oder ein Fluoratom bedeutet.

2. Benzoylharnstoff-Verbindung nach Anspruch 1, in der R ein Wasserstoffatom bedeutet.

3. Benzoylharnstoff-Verbindung nach Anspruch 1, in der R ein Fluoratom bedeutet.

4. Verfahren zur Herstellung einer Benzoylharnstoff-Verbindung der Formel

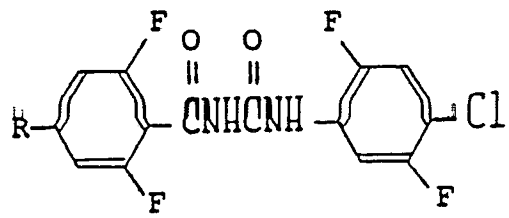

in der R ein Wasserstoff- oder ein Fluoratom bedeutet, umfassend die Umsetzung einer Benzoylisocyanat-Verbindung der Formel

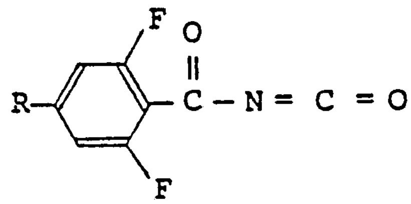

in der R wie vorstehend definiert ist, mit einer Anilin-Verbindung der Formel

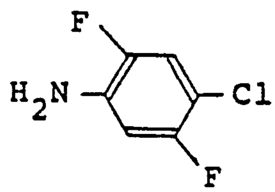

5. Verfahren zur Herstellung einer Benzoylharnstoff-Verbindung der Formel

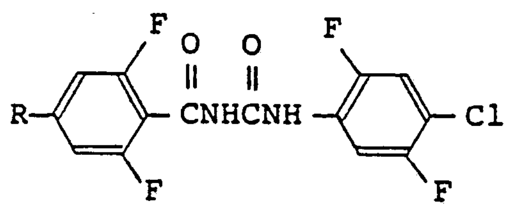

in der R ein Wasserstoff- oder ein Fluoratom bedeutet, umfassend die Umsetzung einer Benzamid-Verbindung der Formel

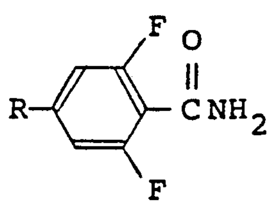

in der R wie vorstehend definiert ist, mit einer Isocyanat-Verbindung der Formel

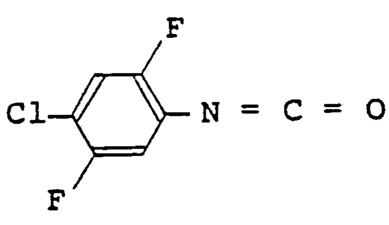

6. Insektizides Mittel, umfassend eine Benzoylharnstoff-Verbindung nach Anspruch 1 und einen inerten Träger oder Verdünnungsmittel.

7. Insektizides Mittel nach Anspruch 6, in dem die Benzoylharnstoff-Verbindung die Formel

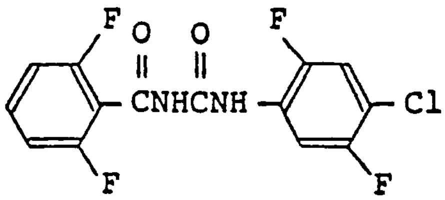

aufweist.

8. Insektizides Mittel nach Anspruch 6, in dem die Benzoylharnstoff-Verbindung die Formel

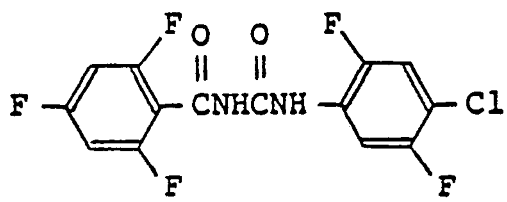

aufweist.

9. Verfahren zur Kontrolle oder Vernichtung von Insekten, umfassend die Anwendung einer insektizid wirksamen Menge der Benzoylharnstoff-Verbindung nach Anspruch 1 auf die Insekten.

10. Verwendung eines insektiziden Mittels nach Anspruch 6 als Insektizid.

**Revendications**

1. Dérivé de benzoylurée représenté par la formule

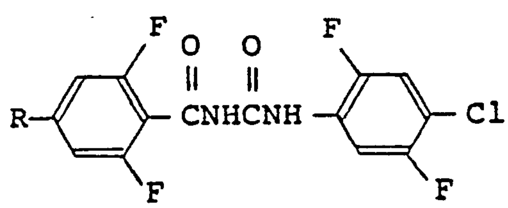

dans laquelle R est un atome d'hydrogène ou de fluor.

2. Dérivé de benzoylurée selon la revendication 1, dans lequel R est un atome d'hydrogène.

3. Dérivé de benzoylurée selon la revendication 1, dans lequel R est un atome de fluor.

4. Procédé de production d'un dérivé de benzoylurée représenté par la formule

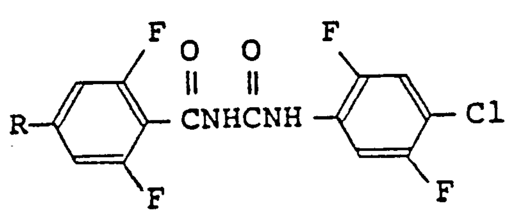

dans laquelle R est un atome d'hydrogène ou de fluor, qui consiste à faire réagir un dérivé de benzoylisocyanate représenté par la formule

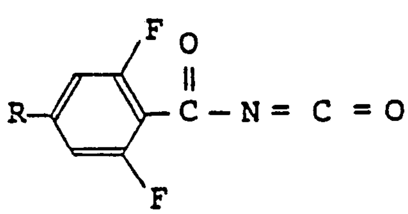

dans laquelle R est tel que défini précédemment, avec un dérivé d'aniline représenté par le formule

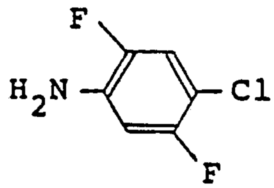

5. Procédé de production d'un dérivé de benzoylurée représenté par la formule

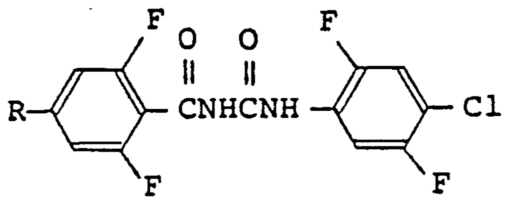

dans laquelle R est un atome d'hydrogène ou de fluor, qui consiste à faire réagir un dérivé de benzamide représenté par la formule

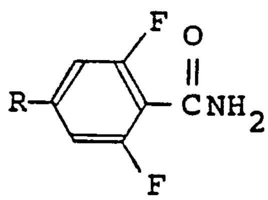

dans laquelle R est tel que défini précédemment, avec un dérivé d'isocyanate représenté par la formule

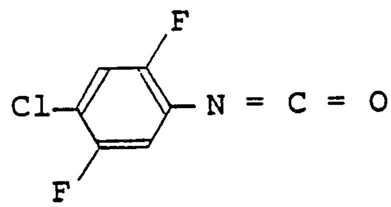

6. Composition insecticide qui comprend un dérivé de benzoylurée selon la revendication 1 et un support ou diluant inerte.

7. Composition insecticide selon la revendication 6, dans laquelle le dérivé de benzoylurée est représenté par la formule

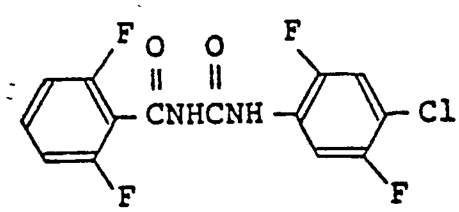

8. Composition insecticide selon la revendication 6, dans laquelle le dérivé de benzoylurée est représenté par la formule

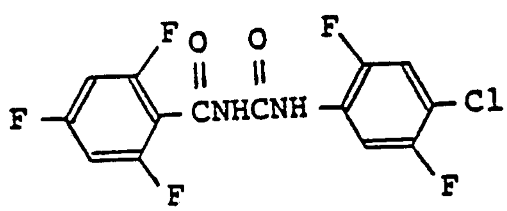

9. Procédé de lutte contre les insectes ou pour leur extermination qui consiste à appliquer aux insectes une quantité efficace sur le plan insecticide d'un dérivé de benzoylurée selon la revendication 1.

10. Utilisation d'une composition insecticide selon la revendication 6 en tant qu'insecticide.